# EUROPÄISCHE PATENTSCHRIFT

(11) **EP 0 640 013 B1**
(45) Veröffentlichungstag und Bekanntmachung des Hinweises auf die Patenterteilung: **18.09.1996**
(21) Anmeldenummer: 93911792.5
(22) Anmeldetag: 03.05.1993
(51) Int. Cl.: B01J 31/02, B01J 31/04, C07H 5/04, C07H 15/04

(54) **SAURE KATALYSATOREN**
ACID CATALYSTS
CATALYSEURS ACIDES

(30) Priorität: 12.05.1992 DE 4215558
(43) Veröffentlichungstag der Anmeldung: 01.03.1995
(73) Patentinhaber: Henkel Kommanditgesellschaft auf Aktien, 40191 Düsseldorf (DE)
(72) Erfinder: FABRY, Bernd, D-4052 Korschenbroich (DE); WEUTHEN, Manfred, D-5650 Solingen 11 (DE); WANGEMANN, Frank, D-5650 Solingen 11 (DE)
(86) Internationale Anmeldenummer: EP9301058
(87) Internationale Veröffentlichungsnummer: WO9323163

(56) Entgegenhaltungen:
- EP-A- 0 178 557
- WO-A-90/06933
- US-A- 4 795 728
- US-A- 4 950 743

## Beschreibung

### Gebiet der Erfindung

Die Erfindung betrifft saure Katalysatoren, ein verfahren zu ihrer Herstellung durch Sulfierung kurzkettiger Triglyceride sowie ihre Verwendung zur Herstellung von Alkyloligoglucosiden, Fettsäureestern und Fettalkoholpolyalkylenglycolethern.

### Stand der Technik

Alkyloligoglykoside, insbesondere Alkyloligoglucoside, stellen nichtionische Tenside dar, die infolge ihrer vielfältigen anwendungstechnischen Eigenschaften und ihrer ausgezeichneten ökotoxikologischen Verträglichkeit zunehmend an Bedeutung gewinnen.

Zu ihrer Herstellung geht man üblicherweise von Zuckern oder wäßrigen Stärkeabbauprodukten aus, die - gegebenenfalls über die Zwischenstufe der Butylglykoside - mit primären Alkoholen acetalisiert werden. Die Kondensation macht gewöhnlich die Anwesenheit saurer Katalysatoren erforderlich, die in der Patentliteratur bereits umfangreich beschrieben worden sind.

In der Patentschrift **EP-B1-0 132 043** (Procter & Gramble) wird beispielsweise vorgeschlagen, die Acetalisierung von Glucose und Fettalkohol im Gegenwart tensidischer Katalysatoren durchzuführen, die man beispielsweise durch Umsetzung von Fettalkoholen oder Alkylbenzolen mit Schwefeltrioxid erhält. In ähnlicher Weise beschreibt die Patentschrift **US 5,003,057** (Henkel Corp.) die Verwendung von Naphthalinsulfonsäure. Von Nachteil ist hierbei jedoch, daß die genannten Katalysatoren während der Acetalisierung zu einer starken Schaumentwicklung beitragen können, die eine Verminderung des nutzbaren Reaktorvolumens und damit der Raum-Zeit-Ausbeute zur Folge hat.

Aus der Offenlegungsschrift **EP-A-0 415 192** ist des weiteren die Acetalisierung von Zuckern mit Alkoholen in Gegenwart von Sulfobernsteinsäure bekannt. Für die Herstellung von Alkyloligoglycosiden ist dieser Katalysator jedoch ebenfalls nicht zufriedenstellend, da die Herstellung mit hohem technischen Aufwand verbunden ist.

Die Aufgabe der Erfindung bestand somit darin, verbesserte saure Katalysatoren zu entwickeln, die frei von den geschilderten Nachteilen sind.

### Beschreibung der Erfindung

Gegenstand der Erfindung sind saure Katalysatoren, die man dadurch erhält, daß man kurzkettige Triglyceride der Formel **(I)**, 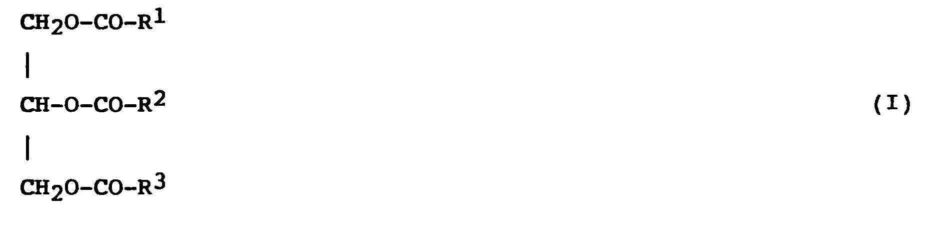 in der R¹, R² und R³ unabhängig für Alkylreste mit 1 bis 4 Kohlenstoffatomen stehen, mit Schwefeltrioxid umsetzt.

Überraschenderweise wurde gefunden, daß "Sulfotriglyceride" der genannten Art ausgezeichnete saure Katalysatoren für eine Vielzahl von Reaktionen darstellen. Sie sind beispielsweise in Fettalkoholen spontan und klar löslich, niedrigviskos und daher leicht dosierbar und weisen zudem den Vorteil einer leichten Zugänglichkeit und problemlosen Herstellbarkeit auf.

Ein weiterer Gegenstand der Erfindung betrifft ein Verfahren zur Herstellung saurer Katalysatoren, bei dem man kurzkettige Triglyceride der Formel **(I)**, 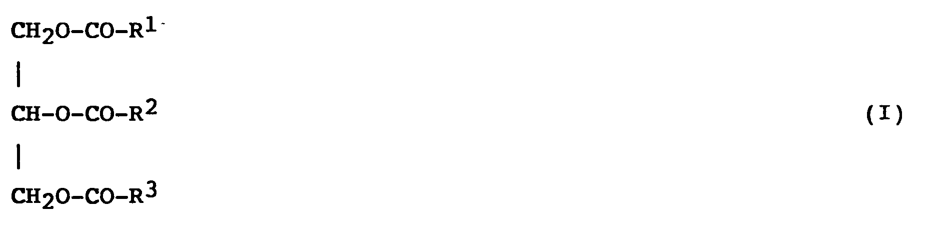 in der
R¹, R² und R³ unabhängig voneinander für Alkylreste mit 1 bis 4 Kohlenstoffatomen stehen, mit Schwefeltrioxid umsetzt.

Typische Beispiele für kurzkettige Triglyceride, die im Sinne des erfindungsgemäßen Verfahrens als Ausgangsstoffe in Betracht kommen, sind Glycerintripropionat, Glycerintributyrat und Glycerintrivaleriat. Infolge der leichten Zugänglichkeit sowie der hohen katalytischen Aktivität des Sulfierproduktes, ist die Verwendung von Glycerintriacetat bevorzugt; in gleicher Weise bevorzugt sind demzufolge die Sulfierprodukte des Glycerintriacetats als saure Katalysatoren.

Die kurzkettigen Triglyceride und das Schwefeltrioxid können im molaren Verhältnis von 1 : 0,5 bis 1 : 3,3 eingesetzt werden. Als besonders aktiv haben sich saure Katalysatoren erwiesen, die durch Umsetzung der kurzkettigen Triglyceride mit Schwefeltrioxid im molaren Verhältnis von 1 : 1 bis 1 : 3, insbesondere 1 : 2 bis 1 : 3 erhalten werden.

Die Sulfiertemperatur ist in weiten Grenzen unkritisch und kann zwischen 10 und 80°C betragen. Ein besonderer Vorteil des erfindungsgemäßen Verfahrens besteht jedoch in der überraschenden Erkenntnis, daß eine praktisch vollständige Aufnahme des Schwefeltrioxids durch die eingesetzten Triglyceride bereits bei vergleichsweise niedrigen Temperaturen von 15 bis 40, insbesondere 20 bis 35 °C, stattfindet. Durch diese schonenden Bedingungen werden Sulfierprodukte erhalten, die ohne Bleiche hellfarbig sind.

Die Sulfierung kann in einer Weise erfolgen, wie sie beispielsweise von der Umsetzung von Methylestern mit Schwefeltrioxid bekannt und umfangreich dokumentiert worden ist [vgl. **J. Falbe (ed.) "Surfactants in consumer products", Springer Verlag, Berlin, 1987, S.54-85].** Des weiteren sei auf die Offenlegungsschriften **WO 91/06532** und **DE-A-39 41 365** (Henkel KGaA) verwiesen, die die Sulfierung von gesättigten bzw. ungesättigten langkettigen Triglyceriden zum Gegenstand haben. Die Reaktion kann batchweise durchgeführt werden, vorzugsweise erfolgt die Sulfierung jedoch kontinuierlich in einem Fallfilmreaktor. Das eingesetzte Schwefeltrioxid wird dabei in an sich bekannter Weise mit einem Inertgas, beispielsweise Stickstoff oder Luft, auf einen Wert von 1 bis 10, vorzugsweise 3 bis 5 Vol.-% verdünnt.

Die Sulfierung der kurzkettigen Triglyceride führt zu einer komplexen Mischung von Produkten, in der überwiegend sulfatierte Partialglyceride und Sulfocarbonsäuren enthalten sind. Die Zusammensetzung wird dabei im wesentlichen durch das Einsatzverhältnis von Triglycerid und SO₃ bestimmt.

### Gewerbliche Anwendbarkeit

Ein weiterer Gegenstand der Erfindung betrifft die Verwendung der sauren Katalysatoren in der Acetalisierung von Zuckern mit Fettalkoholen zur Herstellung von Alkyl- und/oder Alkenyloligoglykosiden der Formel **(II)**, 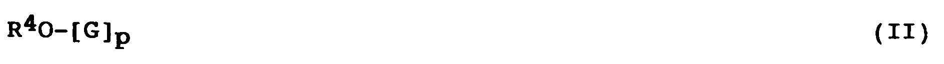 in der
- R⁴: für einen Alkyl- und/oder Alkenylrest mit 1 bis 22 Kohlenstoffatomen
- G: für einen Zuckerrest mit 5 oder 6 Kohlenstoffatomen und
- p: für Zahlen von 1 bis 10
steht.

Ein weiterer Gegenstand der Erfindung betrifft die Verwendung der sauren Katalysatoren in der Veresterung von Fettsäuren mit Alkoholen zur Herstellung von Fettsäureestern der Formel **(III)**, 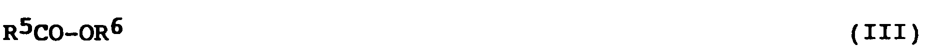 in der
- R⁵CO: für einen Acylrest mit 6 bis 22 Kohlenstoffatomen und O oder 1 bis 5 Doppelbindungen und
- R⁶: für einen linearen oder verzweigten Alkyl- und/oder Alkenylrest mit 1 bis 22 Kohlenstoffatomen
steht.

Ein weiterer Gegenstand der Erfindung betrifft schließlich die Verwendung der sauren Katalysatoren in der Alkoxylierung von Fettalkoholen mit Alkylenoxiden zur Herstellung von Fettalkoholpolyalkylenglycolethern der Formel **(IV)**, 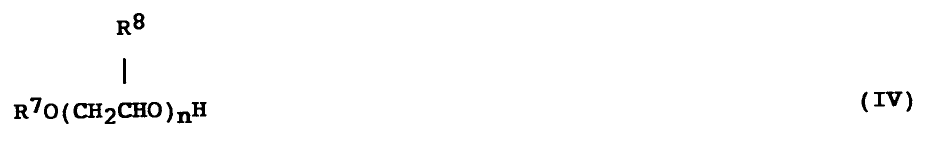 in der
- R⁷: für einen Alkyl- und/oder Alkenylrest mit 6 bis 22 Kohlenstoffatomen
- R⁸: für Wasserstoff oder einen Methylrest und
- n: für Zahlen von 1 bis 30
steht.

Die sauren Katalysatoren können in den geschilderten Verfahren in Mengen von 0,5 bis 10, vorzugsweise 1 bis 3 Gew.-% - bezogen auf die Ausgangsstoffe - eingesetzt werden.

Die folgenden Beispiele sollen den Gegenstand der Erfindung näher erläutern, ohne ihn darauf einzuschränken.

### Beispiele

### I. Herstellungsbeispiele

### Beispiel 1:

**Herstellang von Sulfotriacetin-I.** In einem 500-ml-Sulfierreaktor mit Gaseinleitungsrohr und Mantelkühlung wurden 218 g (1 mol) Glycerintriacetat ("Triacetin", Verkaufsprodukt Fa. Henkel KGaA, Düsseldorf, FRG) vorgelegt und über einen Zeitraum von 30 min mit 80 g (1 mol) Schwefeltrioxid umgesetzt. Das SO₃ war zuvor aus einer entsprechenden Menge 85 gew.-%igem Oleums ausgetrieben und mit Stickstoff auf eine Konzentration von 5 Vol-% verdünnt worden. Das Sulfotriacetin-I wurde in praktisch quantitativer Ausbeute in Form einer farblosen, leicht beweglichen Flüssigkeit gewonnen.

### Beispiel 2:

**Herstellung von Sulfotriacetin-II.** Beispiel 1 wurde unter Einsatz von 218 g Glycerinacetat und 160 g (2 mol) Schwefeltrioxid wiederholt. Die Reaktionszeit betrug 1 h, wobei 92 % des angebotenen SO₃'s aufgenommen wurden. Das resultierende Sulfotriacetin-II wurde ebenfalls als farblose, niedrigviskose Flüssigkeit erhalten.

### Beispiel 3:

**Kontinuierliche Herstellung von Sulfotriacetin-III.** In einem kontinuierlich arbeitenden Fallfilmreaktor (Länge 120 cm, Durchmesser 1 cm, Durchsatz 600 g/h) mit Kühlmantel wurden Glycerintriacetat und Schwefeltrioxid im molaren Verhältnis 1 : 3 zur Reaktion gebracht; das SO₃ wurde wiederum als 5 vol-%ige Mischung mit Stickstoff eingesetzt. Das Sulfotriacetin-III wurde als leicht gelb gefärbte, viskose, jedoch bewegliche Flüssigkeit erhalten.

### II. Anwendungstechnische Beispiele

### Beispiel 4:

**Herstellung von C**_{**12/14**}**-Kokosalkylglucosid.** In einem 1-l-Dreihalskolben mit Rührer, Tropftrichter und Destillationsaufsatz wurden 234 g (1,3 mol) wasserfreie Glucose vorgelegt und mit 1400 g (6,5 mol) C_{12/14} Kokosfettalkohol (Lorol^{(R)} Spezial, Hydroxyzahl: 290; Fa. Henkel KGaA, Düsseldorf, FRG) versetzt. Die Reaktionsmischung wurde auf 90 °C vorgeheizt, ein Vakuum von 20 mbar angelegt und dann über den Tropftrichter innerhalb von 5 min eine Lösung von 0,5 g - entsprechend 0,3 Gew.-% bezogen auf Glucose - von Sulfotriacetin-III in 15 g C_{12/14}- Kokosfettalkohol zudosiert. Nach Beendigung der Zugabe wurde die Reaktionsmischung so lange auf 115 °C erhitzt, bis kein weiteres Kondensationswasser mehr abdestilliert werden konnte. Das rohe Reaktionsprodukt wurde neutralisiert, in eine Vakuumdestillationsapparatur überführt und der überschüssige Fettalkohol bei einer Temperatur von 180 °C und einem verminderten Druck von 5 mbar abdestilliert.

| Kenndaten des Produktes: | |
|---|---|
| Durchschnittlicher Polymerisationsgrad (DP) | 1,3 |
| Polyglucosegehalt | 4,1 Gew.-% |
| Fettalkoholgehalt | 0,8 Gew.-% |

### Beispiel 5:

**Herstellung von C**_{**16/18**}**-Talgfettsäure-2-Ethylhexylester.** In einem 1,5-l-Dreihalskolben mit Rührer und Destillationsaufsatz wurden 280 g (1 mol) C_{16/18}-Talgfettsäure (Edenor^{(R)} HT, Fa. Henkel KGaA, Düsseldorf, FRG) und 730 g (5 mol) 2-Ethylhexanol vorgelegt und mit 8,5 g - entsprechend 3 Gew.-% bezogen auf die Fettsäure - Sulfotriacetin-III versetzt. Die Reaktionsmischung wurde über einen Zeitraum von 2,5 h unter Rückfluß gehalten, bis kein Reaktionswasser mehr abdestilliert werden konnte. Anschließend wurde das rohe Veresterungsprodukt neutralisiert, in eine Vakuumdestillationsapparatur überführt und überschüssiges 2-Ethylhexanol abgetrennt.

| Kenndaten des Produktes | |
|---|---|
| Säurezahl | = < 1 |
| Hydroxyzahl | = < 1 |
| Verseifungszahl | = 135 |

### Beispiel 6:

**Herstellung von C**_{**12/14**}**-Kokosfettalkohol-2EO-ether.** In einem 1-l-Autoklaven wurden 218 g (1 mol) C_{12/14}-Kokosfettalkohol vorgelegt und mit 2 g - entsprechend 1 Gew.-% bezogen auf den Fettalkohol - Sulfotriacetin-I versetzt. Der Autoklav wurde verschlossen, und dreimal abwechselnd mit Stickstoff beaufschlagt und evakuiert. Danach wurde die Reaktionsmischung auf 175 °C erhitzt und portionsweise mit 88 g (2 mol) Ethylenoxid beaufschlagt, wobei der Druck bis auf 4,5 bar anstieg. Nach Beendigung der Zugabe wurde die Reaktionsmischung über 30 min einer Nachreaktion unterworfen, abgekühlt und entspannt. Das rohe Alkoxylierungsprodukt wurde mit Mg(OH)₂ auf pH = 7,5 eingestellt.

| Kenndaten des Produktes | |
|---|---|
| Hydroxylzahl (bestimmt) | 179 |
| Hydroxylzahl (Theorie) | 183 |

## Patentansprüche

1. Saure Katalysatoren dadurch erhältlich, daß man kurzkettige Triglyceride der Formel **(I)**, 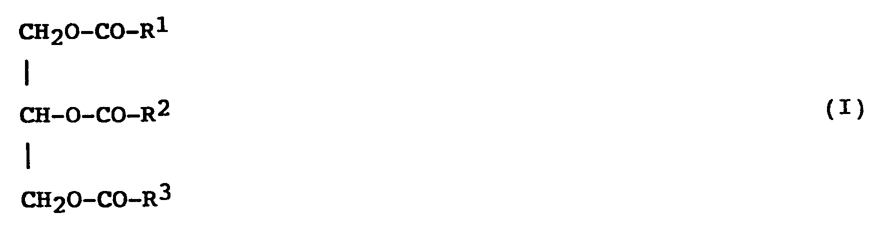 in der
R¹, R² und R³ unabhängig voneinander für Alkylreste mit 1 bis 4 Kohlenstoffatomen stehen, mit Schwefeltrioxid umsetzt.

2. Verfahren zur Herstellung saurer Katalysatoren, bei dem man kurzkettige Triglyceride der Formel **(I)**, 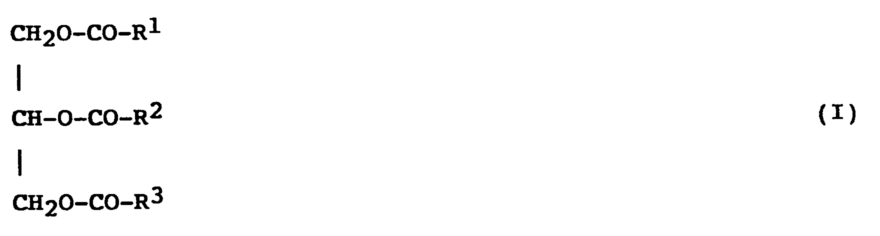 in der
R¹, R² und R³ unabhängig voneinander für Alkylreste mit 1 bis 4 Kohlenstoffatomen stehen, mit Schwefeltrioxid umsetzt.

3. Verfahren nach Anspruch 2, **dadurch gekennzeichnet,** daß man Triglyceride der Formel (I) einsetzt, in der R¹, R² und R³ für Methylreste stehen.

4. Verfahren nach Anspruch 2, **dadurch gekennzeichnet,** daß man die Triglyceride und das Schwefeltrioxid im molaren Verhältnis von 1 : 0,5 bis 1 : 3,3 einsetzt.

5. Verfahren nach Anspruch 2, **dadurch gekennzeichnet**, daß man die Reaktion bei 10 bis 80 °C durchführt.

6. Verfahren nach Anspruch 2, **dadurch gekennzeichnet,** daß man die Reaktion kontinuierlich in einem Fallfilmreaktor durchführt.

7. Verwendung von sauren Katalysatoren nach Anspruch 1 zur Herstellung von Alkyl- und/oder Alkenyloligoglycosiden der Formel (II), 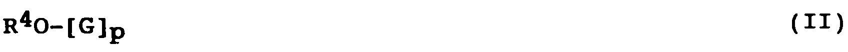 in der
R⁴ für einen Alkyl- und/oder Alkenylrest mit 1 bis 22 Kohlenstoffatomen,
G für einen Zuckerrest mit 5 oder 6 Kohlenstoffatomen und
p für Zahlen von 1 bis 10
steht.

8. Verwendung von sauren Katalysatoren nach Anspruch 1 zur Herstellung von Fettsäureestern der Formel **(III)**, 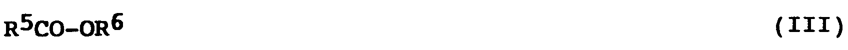 in der
R⁵CO für einen Acylrest mit 6 bis 22 Kohlenstoffatomen und O oder 1 bis 5 Doppelbindungen und
R⁶ für einen linearen oder verzweigten Alkyl- und/oder Alkenylrest mit 1 bis 22 Kohlenstoffatomen
steht.

9. Verwendung von sauren Katalysatoren nach Anspruch 1 zur Herstellung von Fettalkoholpolyalkylenglycolethern der Formel **(IV)**, 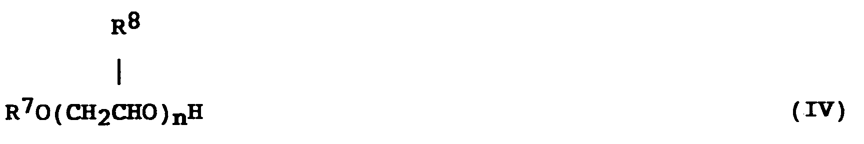 in der
R⁷ für einen Alkyl- und/oder Alkenylrest mit 6 bis 22 Kohlenstoffatomen,
R⁸ für Wasserstoff oder einen Methylrest und
n für Zahlen von 1 bis 30
steht.

## Claims

1. Acidic catalysts obtainable by reaction of short-chain triglycerides corresponding to formula **(I)** 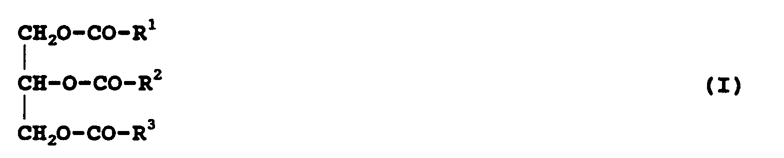 in which R¹, R² and R³ independently of one another represent alkyl radicals containing 1 to 4 carbon atoms, with sulfur trioxide.

2. A process for the production of acidic catalysts, in which short-chain triglycerides corresponding to formula **(I)** 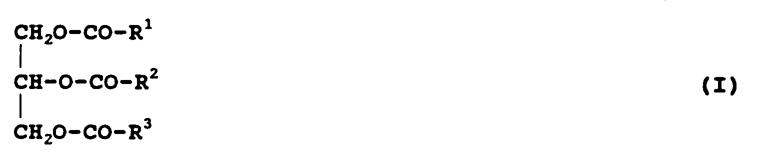 in which R¹, R² and R³ independently of one another represent alkyl radicals containing 1 to 4 carbon atoms, are reacted with sulfur trioxide.

3. A process as claimed in claim 2, **characterized in that** triglycerides corresponding to formula **(I)**, in which R¹, R² and R³ are methyl radicals, are used.

4. A process as claimed in claim 2, **characterized in that** the triglycerides and the sulfur trioxide are used in a molar ratio of 1:0.5 to 1:3.3.

5. A process as claimed in claim 2, **characterized in that** the reaction is carried out at 10 to 80°C.

6. A process as claimed in claim 2, **characterized in that** the reaction is carried out continuously in a falling-film reactor.

7. The use of the acidic catalysts claimed in claim 1 for the production of alkyl and/or alkenyl oligoglycosides corresponding to formula **(II)** 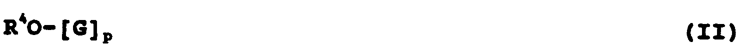 in which
R⁴ is an alkyl and/or alkenyl radical containing 1 to 22 carbon atoms,
G is a sugar unit containing 5 or 6 carbon atoms and
p is a number of 1 to 10.

8. The use of the acidic catalysts claimed in claim 1 for the production of fatty acid esters corresponding to formula **(III)** 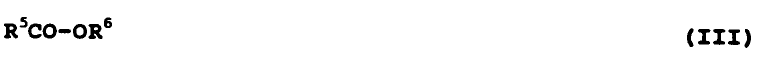 in which
R⁵CO is an acyl radical containing 6 to 22 carbon atoms and 0 or 1 to 5 double bonds and
R⁶ is a linear or branched alkyl and/or alkenyl radical containing 1 to 22 carbon atoms.

9. The use of the acidic catalysts claimed in claim 1 for the production of fatty alcohol polyalkylene glycol ethers corresponding to formula **(IV)** 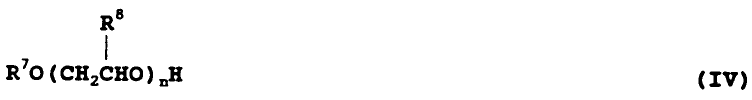 in which
R⁷ is an alkyl and/or alkenyl radical containing 6 to 22 carbon atoms,
R⁸ is hydrogen or a methyl radical and
n is a number of 1 to 30.

## Revendications

1. Catalyseurs acides que l'on obtient, en faisant réagir avec l'anhydride sulfurique des triglycérides à chaînes courtes de la formule (I), 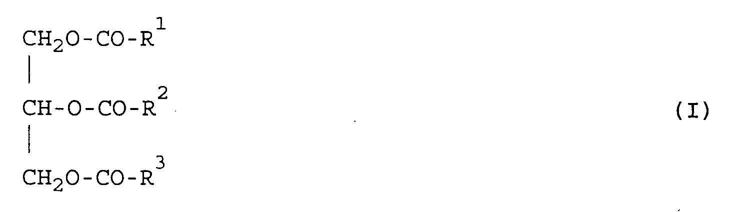 dans laquelle R¹, R² et R³ représentent indépendamment l'un de l'autre des radicaux alkyle ayant de 1 à 4 atomes de carbone.

2. Procédé de préparation de catalyseurs acides, dans lequel on fait réagir avec de l'anhydride sulfurique des triglycérides à chaîne courte et de formule (I), 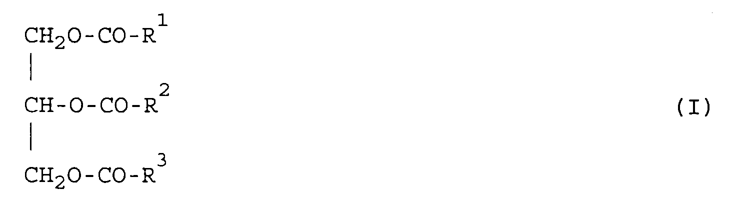 dans laquelle R¹, R² et R³ représentent indépendamment l'un de l'autre des radicaux alkyle ayant de 1 à 4 atomes de carbone.

3. Procédé selon la revendication 2,
caractérisé en ce que
on met en jeu des triglycérides de la formule (I), dans laquelle R¹, R² et R³ représentent des radicaux méthyle.

4. Procédé selon la revendication 2,
caractérisé en ce que
on met en oeuvre les triglycérides et l'anhydride sulfurique dans le rapport molaire de 1:0,5 à 1:3,3.

5. Procédé selon la revendication 2,
caractérisé en ce que
on réalise la réaction entre 10 et 80°C.

6. Procédé selon la revendication 2,
caractérisé en ce que
on réalise la réaction en continu dans un réacteur à ruissellement.

7. Utilisation de catalyseurs acides selon la revendication 1 pour la préparation d'alkylglycosides et/ou d'alcényloligoglycosides de la formule (II),
R⁴O-[G]ₚ (II)
dans laquelle
R⁴ représente un radical alkyle et/ou un radical alcényle avec de 1 à 22 atomes de carbone,
G représente un radical sucre avec 5 ou 6 atomes de carbone et,
p représente des nombres de 1 à 10.

8. Utilisation de catalyseurs acides selon la revendication 1 pour la préparation d'esters d'acide gras de la formule (III),
R⁵CO-OR⁶
dans laquelle
R⁵CO représente un radical acyle ayant de 6 à 22 atomes de carbone et O ou 1 à 5 double liaisons et,
R⁶ représente un radical alkyle et/ou un radical alcényle avec de 1 à 22 atomes de carbone.

9. Utilisation de catalyseurs acides selon la revendication 1 pour la production d'éthers d'alcool gras de polyalkylèneglycol de la formule (IV), 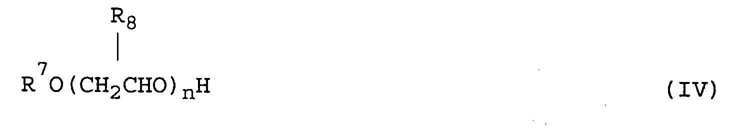 dans laquelle
R⁷ représente un radical alkyle et/ou un radical alcényle ayant de 6 à 22 atomes de carbone,
R⁸ représente de l'hydrogène ou un radical méthyle et, n représente des nombres de 1 à 30.
